# EUROPÄISCHE PATENTSCHRIFT

(11) **EP 0 850 876 B1**
(45) Veröffentlichungstag und Bekanntmachung des Hinweises auf die Patenterteilung: **29.12.1999**
(21) Anmeldenummer: 97120682.6
(22) Anmeldetag: 26.11.1997
(51) Int. Cl.: C01B 13/20, C01B 31/18, B01J 19/24, B01J 19/26, B01J 12/02

(54) **Mittels Aerosol dotierte, pyrogen hergestellte Oxide**
Doped oxides prepared pyrogenically using an aerosol
Préparation pyrogénique d'oxydes dopés utilisant un aérosol

(30) Priorität: 05.12.1996 DE 19650500
(43) Veröffentlichungstag der Anmeldung: 01.07.1998
(73) Patentinhaber: Degussa-Hüls Aktiengesellschaft, 60287 Frankfurt am Main (DE)
(72) Erfinder: Mangold, Helmut, Dr., 63517 Rodenbach (DE); Golchert, Rainer, 64283 Darmstadt (DE); Katusic, Stipan, 65779 Kelkheim (DE); Janzon, Karlheinz, Dr., 63571 Gelnhausen-Hailer (DE)

(56) Entgegenhaltungen:
- EP-A- 0 023 587
- EP-A- 0 241 647
- CH-A- 381 205
- NL-C- 95 381
- US-A- 4 259 310
- US-A- 4 292 290
- FORMENTI M. ET AL.: "Preparation in a hydrogen-oxygen flame of ultrafine metal oxide particles" JOURNAL OF COLLOID AND INTERFACE SCIENCE, Bd. 39, Nr. 1, April 1972, Seiten 79-89, XP002053084
- DATABASE WPI Section Ch, Derwent Publications Ltd., London, GB; Class E37, AN 73-49486U XP002053085 & JP 48 008 688 A (SHINETSU CHEM IND CO LTD)

## Beschreibung

Die Erfindung betrifft mittels Aerosol dotierte, pyrogen hergestellte Oxide, ein Verfahren zu ihrer Herstellung und ihre Verwendung.

Es ist bekannt, auf pyrogenem Wege Siliciumdioxid-Mischoxide herzustellen, indem man das dampfförmige Chlorid des Siliciums mit den dampfförmigen Chloriden des Eisens, Titans oder Zirkons vermischt und in der Knallgasflamme umsetzt (EP-A 0 023 587).

Es ist bekannt, pyrogen hergestellte Zirkonoxide mit Salzen seltener Erden zu dotieren, indem man eine Suspension des Zirkonoxids mit einer wäßrigen Lösung eines Salzes seltener Erden vermischt und diese Mischung sprühtrocknet (EP-A 0 241 647).

Es ist bekannt, pyrogen hergestelltes Siliciumdioxid zu produzieren, indem man einen Brenner, der aus mehreren unterschiedlich langen Rohren besteht, verwendet. Hierdurch wird das Zusetzen des Brenners mit SiO₂ vermieden (JP-A 48-008688).

Es ist bekannt, Titandisulfid in der Dampfphase aus Titantetrachlorid und Schwefel herzustellen (US-A 4,259,310).

Es ist bekannt, pyrogen hergestellte Oxide mit Metallsalzen oder Metalloxiden zu belegen, indem man die pyrogen hergestellten Oxide mit wäßrigen Lösungen von Metallsalzen vermischt und anschließend trocknet und/oder kalziniert.

Derartig hergestellte Produkte haben den Nachteil, daß a) die Dotierungssubstanz nicht homogen im ganzen Primärpartikel verteilt ist oder, daß b) - je nach Art der Dotierung - Inhomogenitäten in der Mischung auftreten. So können nach der Dotierung und Kalzinierung die Primärpartikel der Dotierungssubstanz separiert und in viel größerem Durchmesser als die Primärpartikel der pyrogenen Oxide vorliegen.

Es bestand somit die Aufgabe pyrogen hergestellte Oxide mit einer weiteren Substanz homogen zu dotieren, und gleichzeitig zu vermeiden, daß neben den Primärpartikeln des pyrogenen hergestellten Oxids separat Primärpartikel der Dotierungssubstanz bzw. Oxide der Dotierungssubstanz vorliegen.

Gegenstand der Erfindung sind mittels Aerosol dotierte, pyrogen hergestellte Oxide von Metallen und/oder Metalloiden, welche dadurch gekennzeichnet sind, daß die Basiskomponente pyrogen, mittels Flammenhydrolyse hergestellte Oxide von Metallen und/oder Metalloiden sind, die mit mindestens einer Dotierungskomponente von 0,00001 bis 20 Gew.-% dotiert sind, wobei die Dotierungsmenge vorzugsweise im Bereich von 1 bis 10.000 ppm liegen kann, und die Dotierungskomponente ein Metalloid und/oder Metall oder ein Metalloidsalz und/oder Metallsalz oder ein Oxid eines Metalls und/oder Metalloids ist, und die BET-Oberfläche der dotierten Oxide zwischen 5 und 600 m²/g liegt.

Ein weiterer Gegenstand der Erfindung ist ein Verfahren zur Herstellung der mittels Aerosol dotierten, pyrogen hergestellten Oxide von Metallen und/oder Metalloiden, welches dadurch gekennzeichnet ist, daß man in eine Flamme, wie sie zur Herstellung von pyrogenen Oxiden mittels Flammenhydrolyse in bekannter Weise benutzt wird, ein Aerosol einspeist, dieses Aerosol vor der Reaktion mit dem Gasgemisch der Flammenoxidation bzw. Flammenhydrolyse homogen mischt, das Aerosol-Gasgemisch in einer Flamme abreagieren läßt und die entstandenen dotierten pyrogen hergestellten Oxide in bekannter Weise vom Gasstrom abtrennt, wobei als Ausgangsprodukt des Aerosols eine Salzlösung oder Suspension, die die Komponente der zu dotierenden Substanz, die ein Metallsalz- oder Metalloidsalz oder Mischungen von beiden oder eine Suspension einer unlöslichen Metall- oder Metalloidverbindung oder einer Mischung aus beiden sein kann, enthält, dient, wobei das Aerosol durch Vernebelung mittels einer Zweistoffdüse oder durch einen Aerosolgenerator vorzugsweise nach der Ultraschallmethode hergestellt wird.

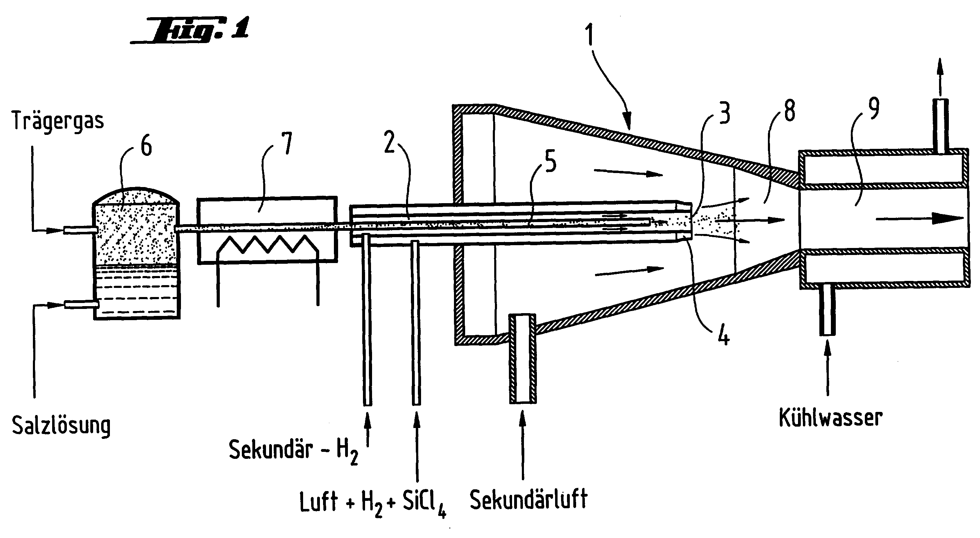

Das Aerosol kann in einer bevorzugten Ausführungsform der Erfindung mittels einer Vorrichtung, wie sie in der Figur 1 dargestellt ist, zugeführt werden. Dabei können für die Gas- und Aerosol-Zufuhr auch die Leitungen vertauscht sein.

In einer weiteren Ausführungsform der Erfindung kann man das Aerosol mittels einer Ringdüse, die in einem beliebigen Winkel, vorzugsweise senkrecht, zum Hauptgasstrom angeordnet ist, zugeführt werden.

Als Basiskomponente können die Metalloide/Metalle Aluminium, Niob, Titan, Wolfram, Zirkon, Germanium, Bor und/oder Silicium eingesetzt werden.

Als Dotierkomponente können Metalle und/oder Metalloide und deren Verbindungen, soweit sie in einer flüssigen Lösung löslich oder suspendierbar sind, eingesetzt werden. In einer bevorzugten Ausführungsform können Verbindungen von Übergangsmetallen und/oder Edelmetallen eingesetzt werden.

Beispielsweise können Cer- und Kaliumsalze als Dotierungskomponenten eingesetzt werden.

Das Verfahren der Flammenhydrolyse zur Herstellung von pyrogenen Oxiden ist aus Ullmanns Enzyklopädie der technischen Chemie, 4. Auflage, Band 21, Seite 464 bekannt.

Durch die feine Verteilung der Dotierungskomponente im Aerosol, sowie die hohen Temperaturen (1.000 bis 2.400 °C) bei der anschließenden Flammenhydrolyse, bei der die Dotierungskomponenten unter Umständen weiter zerkleinert und/oder aufgeschmolzen werden, liegt das Dotierungsmedium während der Genese des pyrogenen Oxids feinverteilt in der Gasphase vor, so daß ein homogener Einbau der Dotierungskomponente in das pyrogene hergestellte Oxid möglich ist.

Mit dem erfindungsgemäßen Verfahren ist es möglich, alle bekannten pyrogen hergestellten Oxide (z.B. SiO₂, TiO₂, Al₂O₃, B₂O₃, ZrO₂, GeO₂, WO₃, Nb₂O₅) mit anderen Oxiden von Metallen oder Metalloiden oder deren Mischungen zu dotieren.

Das erfindungsgemäße Verfahren hat mehrere Vorteile: Durch die Wahl geeigneter Dotierungskomponenten kann man die Aggregat- bzw. Agglomeratstruktur des pyrogenen Oxids beeinflussen.
Weiterhin kann man den pH-Wert des pyrogenen Oxids beeinflussen.

Katalytisch aktive Substanzen (z.B. Cer oder Edelmetalle), die als Dotierungskomponente eingesetzt werden, können nahezu homogen in dem pyrogenen hergestellten Oxid verteilt werden.

Auch die Phasenumwandlung bei pyrogen hergestellten Oxiden, beispielsweise von Rutil zu Anatas im pyrogen hergestellten Titanoxid, kann durch Dotierung beeinflußt werden.

Mit dem erfindungsgemäßen Verfahren können Eigenschaftskombinationen von pyrogen hergestellten Oxiden, die bisher nicht oder nur schwierig d.h. beispielsweise in Verfahren, die mehrere Stufen erfordern, zugänglich waren, erreicht werden.

Die erfindungsgemäß mittels Aerosol dotierten, pyrogen hergestellten Oxide von Metallen und/oder Metalloiden können als Füllstoff, als Trägermaterial, als katalytisch aktive Substanz, als Ausgangsmaterial zur Herstellung von Dispersionen, als Poliermaterial zum Polieren von Metall- bzw- Siliciumscheiben in der Elektroindustrie, als keramischer Grundstoff, in der Elektronikindustrie (CMP-Anwendungen), in der Kosmetikindustrie, als Additiv in der Silikon- und Kautschukindustrie, zur Einstellung der Rheologie von flüssigen Systemen, zur Hitzeschutzstabilisierung, in der Lackindustrie, als Wärmedämmaterial etc. verwendet werden.

Ein weiterer Gegentand der Erfindung ist eine Vorrichtung zur Durchführung des erfindungsgemäßen Verfahrens, welche dadurch gekennzeichnet ist, daß in einem Brenner von für die Herstellung von pyrogenen Oxiden bekannter Bauart für die Zufuhr des Aerosols zusätzlich ein Rohr, vorzugsweise axial, angeordnet ist, wobei das Rohr vor der Düse des Brenners endet.

### Beispiele

Die Brenneranordnung, die in den Beispielen 1 bis 4 verwendet wird, ist in Figur 1 schematisch dargestellt.

Gemäß Figur 1 ist das Kernstück der Apparatur der Brenner 1 bekannter Bauart wie er zur Herstellung von pyrogenen Oxiden üblicherweise verwendet wird.
Der Brenner 1 besteht aus dem Zentralrohr 2, das in die Düse 3, aus welcher der Hauptgasstrom in den Brennerraum 8 strömt und dort abbrennt, mündet. Die innere Düse ist von der weiteren Ringdüse 4 (Manteldüse), aus der zur Vermeidung von Anbackungen Mantel- oder Sekundär-Wasserstoff ausströmt, umgeben.
Erfindungsgemäß befindet sich in dem Zentralrohr 2 das Axialrohr 5, das einige Zentimeter vor der Düse 3 des Zentralrohrs 2 endet. In das Axialrohr 5 wird das Aerosol eingespeist, wobei auf der letzten Stecke des Zentralrohres 2 der Aerosol-Gasstrom des Axialrohres 5 mit dem Gasstrom des Zentralrohres 2 homogen gemischt wird.
Das Aerosol wird in dem Aerosol-Generator 6 (Ultraschallvernebler) erzeugt. Als Aerosoledukt wird eine wäßrige Salzlösung, die das zu dotierende Metall oder Metalloid als Salz in gelöster oder dispergierter/suspendierter Form enthält, verwendet.
Das von dem Aerosol-Generator 6 erzeugte Aerosol wird mittels eines Traggasstromes durch die Heizzone 7, wo das Wasser verdampft und in der Gasphase kleine Salzkristalle in feinverteilter Form zurückbleiben, geleitet.

### Beispiel 1 (ohne Dotierung) 4,44 kg/h SiCl₄ werden bei ca. 130 °C verdampft und in das Zentralrohr des Brenners eingeführt. In das Zentralrohr werden zusätzlich 3 Nm³/h Primär-Wasserstoff und 8,0 Nm³/h Luft eingespeist. Das Gasgemisch strömt aus der inneren Düse des Brenners und brennt in den Brennerraum und das sich daran anschließende wassergekühlte Flammrohr. In die Manteldüse, die die Zentraldüse umgibt, werden zur Vermeidung von Anbackungen an den Düsen 0,5 Nm³/h Mantel- oder Sekundär- Wasserstoff eingespeist. In den Brennerraum werden zusätzlich 12 Nm³/h Sekundär-Luft eingespeist.

Aus dem Axialrohr strömt das Aerosol in das Zentralrohr. Das Aerosol besteht aus Wasserdampf, der durch Ultraschallvernebelung von reinem destilliertem Wasser in dem Aerosolgenerator in einer Menge von 195 g/h erzeugt wird.

Der verhebelte Wasserdampf wird mit Hilfe des Traggases von ca. 0,5 Nm³/h Luft durch eine beheizte Leitung geführt, wobei das Aerosol bei einer Temperatur um 180 °C in Gas übergeht.

Am Brennermund (Düse 3) beträgt die Temperatur des Gasgemisches (SiCl₄-Luft-Wasserstoff, Wasserdampf- bzw. Wasser-Aerosol) 150 °C.

Die Reaktionsgase und die entstandene pyrogene Kieselsäure werden durch Anlegen eines Unterdrucks am Flammrohr 9 durch ein Kühlsystem gesaugt und dabei auf ca. 100 bis 160 °C abgekühlt. In einem Filter oder Zyklon wird der Feststoff von dem Abgasstrom abgetrennt.
Die Kieselsäure fällt als weißes feinteiliges Pulver an. In einem weiteren Schritt werden bei erhöhter Temperatur durch Behandlung mit wasserdampfhaltiger Luft die anhaftenden Salzsäurereste von der Kieselsäure entfernt.

Die BET-Oberfläche der pyrogenen Kieselsäure beträgt 150 m²/g.
Die Herstellparameter sind in der Tabelle 1 zusammengefaßt. Weitere analytische Daten der erhaltenen pyrogenen Kieselsäure sind in der Tabelle 2 angegeben.

### Beispiel 2: (Dotierung mittels Aerosol mit Cer)

Man verfährt wie in Beispiel 1 angegeben:
Es werden 4,44 kg/h SiCl₄ bei ca. 130 °C verdampft und in das Zentralrohr des Brenners eingeführt. In das Zentralrohr werden zusätzlich 3 Nm³/h Primär-Wasserstoff und 8,0 Nm³/h Luft eingespeist. Das Gasgemisch strömt aus der inneren Düse des Brenners und brennt in den Brennerraum und das sich daran anschließende wassergekühlte Flammrohr.
In die Manteldüse, die die Zentraldüse umgibt, werden zur Vermeidung von Anbackungen an den Düsen 0,5 Nm³/h Mantel- oder Sekundär- Wasserstoff eingespeist.
In den Brennerraum werden zusätzlich 12 Nm³/h Sekundär-Luft eingespeist.
Aus dem Axialrohr strömt das Aerosol in das Zentralrohr. Das Aerosol ist ein Cersalz-Aerosol, welches durch Ultraschallvernebelung einer 5 % wäßrigen Cer(III)-Chlorid-Lösung in dem Aerosol-Generator in einer Menge von 210 g/h erzeugt wird.

Das Cersalz-Aerosol wird mit Hilfe des Traggases von 0,5 Nm³/h Luft durch eine beheizte Leitung geführt, wobei das Aerosol bei Temperaturen um ca. 180 °C in ein Gas und ein Salzkristall-Aerosol übergeht.

Am Brennermund beträgt die Temperatur des Gasgemisches (SiCl₄-Luft-Wasserstoff, Aerosol) 180 °C.

Die Reaktionsgase und die entstandene mittels Aerosol mit Cer dotierte, pyrogen hergestellte Kieselsäure werden durch Anlegen eines Unterdrucks durch ein Kühlsystem gesaugt und dabei auf ca. 100 bis 160 °C abgekühlt. In einem Filter oder Zyklon wird der Feststoff von dem Gasstrom abgetrennt.

Die mittels Aerosol dotierte, pyrogen hergestellte Kieselsäure fällt als weißes feinteiliges Pulver an. In einem weiteren Schritt werden bei erhöhter Temperatur durch Behandlung mit wasserdampfhaltiger Luft die anhaftenden Salzsäurereste von der Kieselsäure entfernt.

Die BET-Oberfläche der mittels Aerosol dotierten, pyrogen hergestellten Kieselsäure beträgt 143 m²/g.
Die Herstellparameter sind in der Tabelle 1 zusammengefaßt.
Weitere analytische Daten der erhaltenen mittels Aerosol dotierten pyrogenen Kieselsäure sind in der Tabelle 2 angegeben.

### Beispiel 3 (ohne Dotierung)

4,44 kg/h SiCl₄ werden bei ca. 130 °C verdampft und in das Zentralrohr des Brenners überführt. In das Zentralrohr werden zusätzlich 3 Nm³/h Primär-Wasserstoff und 8,7 Nm³/h Luft eingespeist. Das Gasgemisch strömt aus der inneren Düse des Brenners und brennt in den Brennerraum und das sich daran anschließende wassergekühlte Flammrohr.
In die Manteldüse, die die Zentraldüse umgibt, werden zur Vermeidung von Anbackungen an den Düsen 0,5 Nm³/h Mantel- oder Sekundär- Wasserstoff eingespeist.
In den Brennerraum werden zusätzlich 12 Nm³/h Sekundär-Luft eingespeist.

Aus dem Axialrohr strömt das Aerosol in das Zentralrohr. Das Aerosol besteht aus Wasserdampf, der durch Ultraschallvernebelung von reinem destilliertem Wasser in dem Aerosolgenerator in einer Menge von 210 g/h erzeugt wird.

Das Aerosol wird mit Hilfe des Traggases von ca. 0,5 Nm³/h Luft durch eine beheizte Leitung geführt, wobei das Aerosol bei Temperaturen um ca. 180 °C in Gas übergeht.

Am Brennermund beträgt die Temperatur des Gasgemisches (SiCl₄-Luft-Wasserstoff, Wasserdampf- bzw. Wasser-Aerosol) 180 °C.

Die Reaktionsgase und die entstandene pyrogene Kieselsäure werden durch Anlegen eines Unterdruckes durch ein Kühlsystem gesaugt und dabei auf ca. 100 bis 160 °C abgekühlt. In einem Filter oder Zyklon wird der Feststoff von dem Gasstrom abgetrennt.
Die Kieselsäure fällt als weißes feinteiliges Pulver an. In einem weiteren Schritt werden bei erhöhter Temperatur durch Behandlung mit wasserdampfhaltiger Luft die anhaftenden Salzsäurereste von der Kieselsäure entfernt.

Die BET-Oberfläche der pyrogenen Kieselsäure beträgt 215 m²/g.

Die Herstellparameter sind in der Tabelle 1 zusammengefaßt. Weitere analytische Daten der erhaltenen pyrogenen Kieselsäure sind in der Tabelle 2 angegeben.

### Beispiel 4: (Dotierung mittels Aerosol mit Cer)

Man verfährt wie in Beispiel 1 angegeben:
Es werden 4,44 kg/h SiCl₄ bei ca. 130 °C verdampft und in das Zentralrohr des Brenners eingeführt. In das Zentralrohr werden zusätzlich 3 Nm³/h Primär-Wasserstoff und 8,7 Nm³/h Luft eingespeist. Das Gasgemisch strömt aus der inneren Düse des Brenners und brennt in den Brennerraum und das sich daran anschließende wassergekühlte Flammrohr. In die die Zentraldüse umgebende Manteldüse werden zur Vermeidung von Anbackungen an den Düsen 0,5 Nm³/h Mantel- oder Sekundär-Wasserstoff eingespeist.

In den Brennerraum werden zusätzlich 12 Nm³/h Sekundär-Luft eingespeist.

Aus dem Axialrohr strömt das Aerosol in das Zentralrohr. Das Aerosol ist ein Cersalz-Aerosol, welches durch Ultraschallvernebelung aus einer 5 % wäßrigen Cer(III)-Chlorid-Lösung im Aerosol-Generator in einer Menge von 205 g/h erzeugt wird.

Das Cersalz-Aerosol wird mit Hilfe des Traggases von 0,5 Nm³/h Luft durch eine beheizte Leitung geführt, wobei das Aerosol bei Temperaturen um ca. 180 °C in ein Gas und ein Salzkristall-Aerosol übergeht.

Am Brennermund beträgt die Temperatur des Gasgemisches (SiCl₄-Luft-Wasserstoff, Aerosol) 180 °C.

Die Reaktionsgase und die entstandene mittels Aerosol mit Cer dotierte, pyrogen hergestellte Kieselsäure werden durch Anlegen eines Unterdrucks durch ein Kühlsystem gesaugt und dabei auf ca. 100 bis 160 °C abgekühlt. In einem Filter oder Zyklon wird der Feststoff von dem Gasstrom abgetrennt.

Die mittels Aerosol dotierte, pyrogene Kieselsäure fällt als weißes feinteiliges Pulver an. In einem weiteren Schritt werden bei erhöhter Temperatur durch Behandlung mit wasserdampfhaltiger Luft die anhaftende Salzsäurereste von der mittels Aerosol dotierten pyrogenen Kieselsäure entfernt.

Die BET-Oberfläche der mittels Aerosol dotierten pyrogenen Kieselsäure beträgt 217 m²/g.
Die Herstellparameter sind in der Tabelle 1 zusammengefaßt. Weitere analytische Daten der erhaltenen mittels Aerosol dotierten pyrogenen Kieselsäure sind in der Tabelle 2 angegeben.

### Beispiel 5: (Dotierung mit Kaliumsalzen)

Man verfährt wie in Beispiel 1 beschrieben, wobei als Salzlösung eine 0,5%-wäßrige Kaliumchloridlösung eingesetzt wird.

Es werden 4,44 kg/h SiCl₄ bei ca. 130 °C verdampft und in das Zentralrohr des Brenners eingeführt. In das Zentralrohr werden zusätzlich 3 Nm³/h Primär-Wasserstoff und 8,7 Nm³/h Luft eingespeist. Das Gasgemisch strömt aus der inneren Düse des Brenners und brennt in den Brennerraum und das sich daran anschließende wassergekühlte Flammrohr. In die die Zentraldüse umgebende Manteldüse werden zur Vermeidung von Anbackungen an den Düsen 0,5 Nm³/h Mantel- oder Sekundär- Wasserstoff eingespeist.

Aus dem Axialrohr strömt das Aerosol in das Zentralrohr. Das Aerosol ist ein Kaliumsalz-Aerosol, welches durch Ultraschallvernebelung einer 0,5 % wäßrigen Kaliumchlorid-Lösung in dem Aerosol-Generator in einer Menge von 215 g/h erzeugt wird.

Das Kaliumsalz-Aerosol wird mit Hilfe des Traggases von 0,5 Nm³/h Luft durch eine beheizte Leitung geführt, wobei das Aerosol bei Temperaturen um 180 °C in ein Gas und ein Salzkristall-Aerosol übergeht.

Am Brennermund beträgt die Temperatur des Gasgemisches (SiCl₄-Luft-Wasserstoff, Aerosol) 180 °C.

Die Reaktionsgase und die entstandene mittels Aerosol mit Kalium dotierte, pyrogen hergestellte Kieselsäure werden durch Anlegen eines Unterdrucks durch ein Kühlsystem gesaugt und dabei der Partikel-Gasstrom auf ca. 100 bis 160 °C abgekühlt. In einem Filter oder Zyklon wird der Feststoff von dem Gasstrom abgetrennt.

Die mittels Aerosol dotierte pyrogene Kieselsäure fällt als weißes feinteiliges Pulver an. In einem weiteren Schritt werden bei erhöhter Temperatur durch Behandlung mit wasserdampfhaltiger Luft die anhaftenden Salzsäurereste von der mittels Aerosol dotierten pyrogenen Kieselsäure entfernt.
Die BET-Oberfläche der mittels Aerosol dotierten pyrogenen Kieselsäure beträgt 199 m²/g.
Die Herstellparameter sind in der Tabelle 1 zusammengefaßt. Weitere analytische Daten der erhaltenen mittels Aerosol dotierten pyrogenen Kieselsäure sind in der Tabelle 2 angegeben.

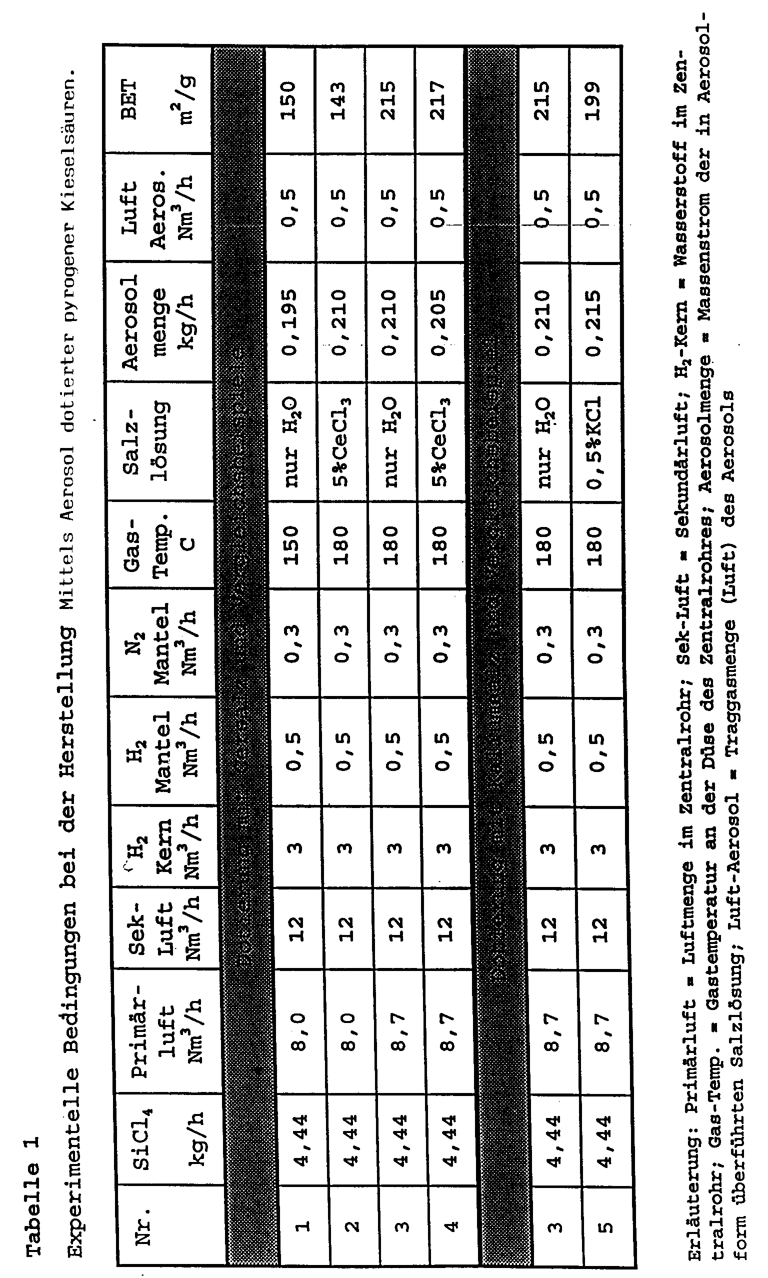

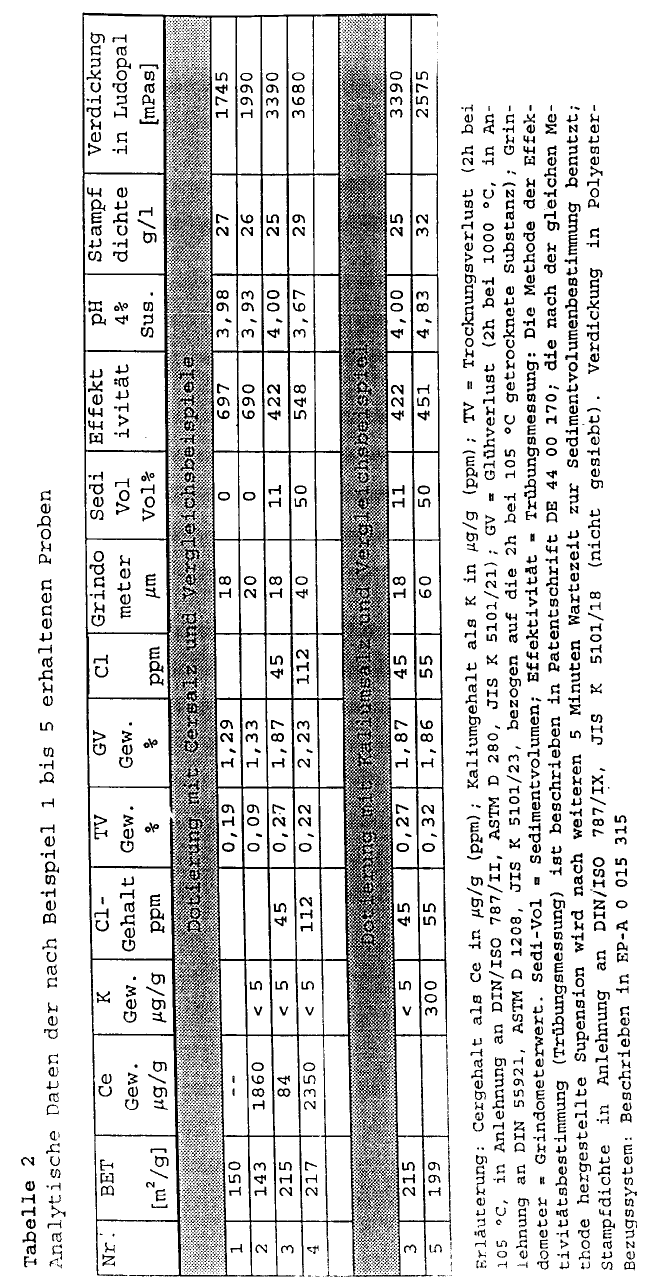

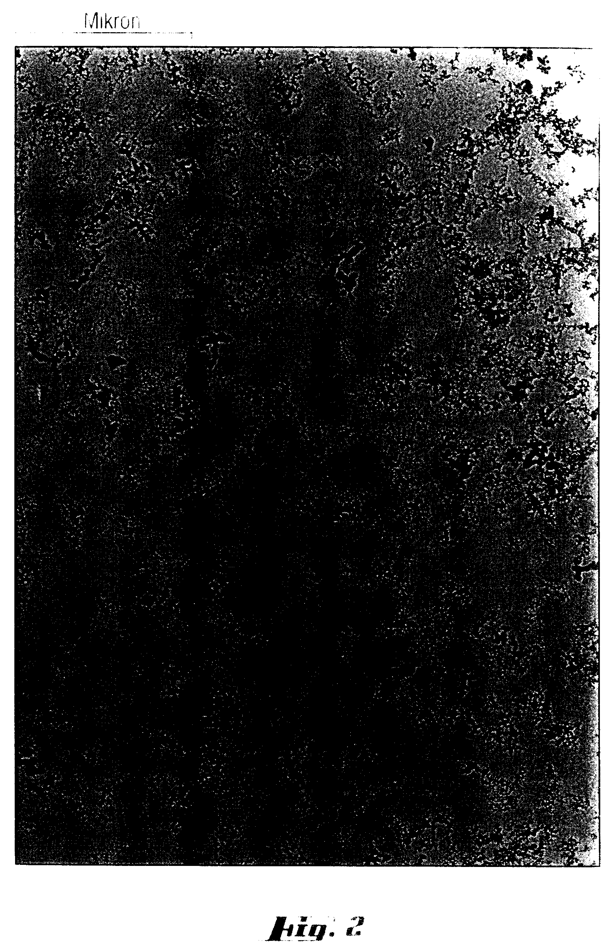

Figur 2 zeigt eine EM-Aufnahme der pyrogenen Kieselsäure, hergestellt nach Beispiel 3 (ohne Dotierung).

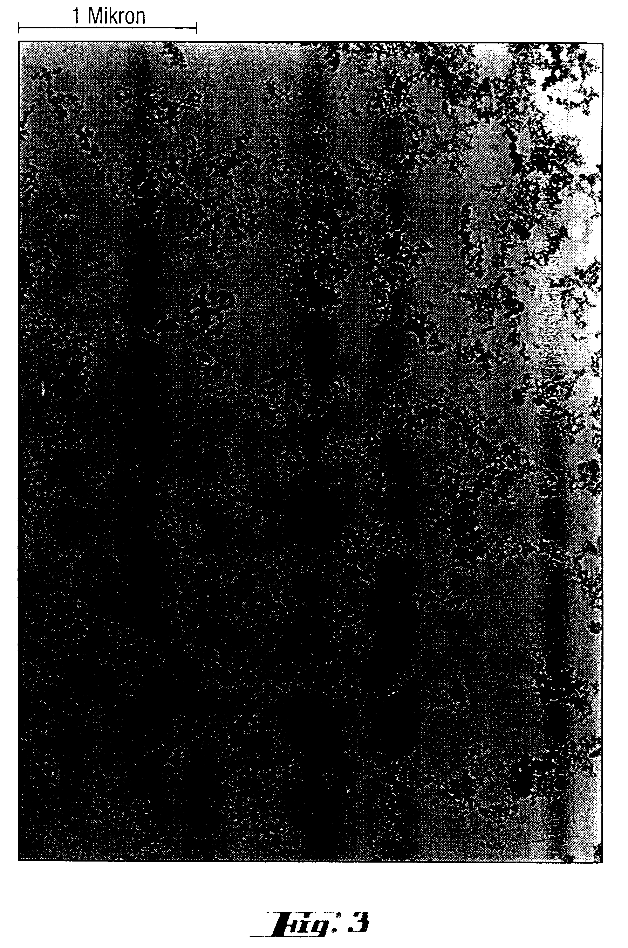

Figur 3 zeigt eine EM-Aufnahme der mittels Aerosol mit Cer dotierten pyrogenen Kieselsäure, hergestellt nach Beispiel 4 (Dotierung mit Cersalz).

Erkennbar ist, daß bei der Dotierung mittels Aerosol mit Cersalz die Aggregat- bzw. Agglomeratstruktur verändert wird. Es entstehen bei der Dotierung mittels Aerosol größer zusammenhängende Strukturen.

Die analytischen Daten der mittels Aerosol dotierten Kieselsäure gemäß Beispiel 4 zeigen gegenüber der Kieselsäure gemäß Beispiel 3 ein erhöhtes Sedimentvolumen und deutlich erhöhte Effektivitätswerte. Dies deutet ebenfalls auf eine Vergrößerung der Aggregat- bzw. Agglomeratstruktur hin.

Weiterhin kann man mit der erfindungsgemäßen, mittels Aerosol mit Cer dotierten Kieselsäure in ungesättigten Polyesterharzen eine deutliche Verbesserung der Verdickungswirkung erzielen.

## Patentansprüche

1. Mittels Aerosol dotierte pyrogen hergestellte Oxide von Metallen und/oder Metalloiden, dadurch gekennzeichnet, daß die Basiskomponente pyrogen, mittels Flammenhydrolyse hergestellte Oxide von Metallen und/oder Metalloiden sind, welche mit mindestens einer Dotierungskomponente von 0,00001 bis 20 Gew.-% dotiert sind, wobei die Dotierungsmenge vorzugsweise im Bereich von 1 bis 10 000 ppm liegen kann, und die Dotierungskomponente ein Metalloid und/oder Metall oder ein Salz oder ein Oxid eines Metalls oder Metalloids ist, und die BET-Oberfläche der dotierten Oxide zwischen 5 und 600 m²/g liegt.

2. Verfahren zur Herstellung von mittels Aerosol dotierten pyrogen hergestellten Oxiden von Metallen und/oder Metalloiden gemäß Anspruch 1, dadurch gekennzeichnet, daß man in eine Flamme, wie sie zur Herstellung von pyrogenen Oxiden mittels Flammenhydrolyse benutzt wird, ein Aerosol einspeist, dieses Aerosol vor der Reaktion mit dem Gasgemisch der Flammenoxidation bzw. Flammenhydrolyse homogen mischt, das Aerosol-Gasgemisch in einer Flamme abreagieren läßt und die entstandenen dotierten pyrogen hergestellten Oxide in bekannter Weise vom Gasstrom abtrennt, wobei als Ausgangsprodukt des Aerosols eine Salzlösung oder Suspension, die die Komponente der zu dotierenden Substanz, die ein Metallsalz- oder Metalloidsalz oder Mischungen von beiden oder eine Suspension einer unlöslichen Metall- oder Metalloidverbindung oder einer Mischung aus beiden sein kann, enthalt, dient, wobei das Aerosol durch Vernebelung mittels einer Zweistoffdüse oder durch einen Aerosolgenerator vorzugsweise nach der Ultraschallvernebelung hergestellt wird.

3. Verwendung der mittels Aerosol dotierten pyrogenen Oxide gemäß Anspruch 1 als Füllstoff, als Trägermaterial, als katalytisch aktive Substanz, als Ausgangsmaterial zur Herstellung von Dispersionen, als Poliermaterial, zum Polieren von Metall- bzw. Siliciumscheiben in der Elektroindustrie, als keramischen Grundstoff, in der Elektronikindustrie (CMP-Anwendungen), in der Kosmetikindustrie, als Additiv in der Silikon- und Kautschukindustrie, zur Einstellung der Rheologie von flüssigen Systemen, zur Hitzestabilisierung, in der Lackindustrie und als Wärmedämmaterial.

4. Vorrichtung zur Durchführung des Verfahren gemäß Anspruch 2, dadurch gekennzeichnet, daß in einem Brenner von für die Herstellung von pyrogenen Oxiden bekannter Bauart für die Zufuhr des Aerosols zusätzlich ein Rohr, vorzugsweise axial, angeordnet ist, wobei das Rohr vor der Düse des Brenners endet.

## Claims

1. Pyrogenically prepared oxides of metals and/or non-metals which are doped using an aerosol, characterised in that the basic components are pyrogenic oxides of metals and/or non-metals prepared by flame hydrolysis, which are doped with at least one doping component at 0.00001 to 20 wt.%, wherein the doping amount may preferably be in the range 1 to 10 000 ppm, and the doping component is a non-metal and/or metal or a salt or an oxide of a metal or non-metal, and the BET surface area of the doped oxides is between 5 and 600 m²/g.

2. A process for preparing pyrogenically prepared oxides of metals and/or non-metals which are doped using an aerosol in accordance with Claim 1, characterised in that an aerosol is fed to a flame such as is used for preparing pyrogenic oxides by flame hydrolysis, this aerosol being homogeneously mixed with the gas mixture for flame oxidation or flame hydrolysis prior to reaction, the aerosol/gas mixture is allowed to react in a flame and the resulting doped pyrogenically prepared oxides are separated from the gas stream in a known manner, wherein a salt solution or suspension which contains the components of the substance to be doped, which may be a metal salt or non-metal salt or mixtures of both or a suspension of an insoluble metal or non-metal compound or a mixture of both, is used as the starting material for the aerosol, wherein the aerosol is produced by nebulisation using a two-fluid nozzle or by an aerosol generator, preferably by ultrasonic nebulisation.

3. Use of pyrogenic oxides which are doped using an aerosol in accordance with Claim 1 as fillers, as support materials, as catalytically active substances, as starting materials for preparing dispersions, as polishing materials to polish metal or silicon wafers in the electrical industry, as ceramic substrates, in the electronics industry (CMP applications), in the cosmetics industry, as additives in the silicone and rubber industry, to adjust the rheology of liquid systems, for heat-resistant stabilisation purposes, in the lacquer industry and as heat insulation materials.

4. Device for performing the process in accordance with Claim 2, characterised in that an additional tube for introducing the aerosol is arranged, preferably axially, in a burner of the structure known for preparing pyrogenic oxides, wherein the tube terminates upstream of the burner nozzle.

## Revendications

1. Oxydes de métaux et de métalloïdes produits de manière pyrogénique, dopés au moyen d'un aérosol,
caractérisés en ce que
• les composants de base sont des oxydes de métaux et/ou de métalloïdes pyrogènes, produits au moyen d'une hydrolyse à la flamme, qui sont dopés avec au moins un composant de dopage de 0,00001 à 20 % en poids, l'importance du dopage pouvant se situer de préférence dans un intervalle de 1 à 10 000 ppm,
• le composant de dopage est un métalloïde et/ou un métal ou un sel et/ou un oxyde d'un métal ou d'un métalloïde, et
• la surface BET des oxydes dopés se situe entre 5 et 600 m²/g.

2. Procédé de production d'oxydes de métaux et/ou de métalloïdes fabriqués de manière pyrogénique et dopés au moyen d'un aérosol selon la revendication 1,
caractérisé en ce qu'
• on introduit dans une flamme un aérosol tel qu'on en utilise pour la production d'oxydes pyrogènes au moyen d'une hydrolyse à la flamme,
• on mélange de manière homogène cet aérosol avant la réaction avec le mélange gazeux de l'oxydation à la flamme ou, selon les cas, de l'hydrolyse à la flamme,
• on laisse réagir le mélange gazeux d'aérosol dans une flamme, et
• on sépare du courant gazeux, de façon connue, les oxydes produits de manière pyrogénique et dopés apparus, et
• on utilise comme produit de départ de l'aérosol une solution ou une suspension de sel qui contient le composant de la substance à doper, qui peut être un sel de métal ou un sel de métalloïde ou des mélanges des deux, ou une suspension d'un composé de métal ou de métalloïde insoluble ou un mélange des deux, l'aérosol étant produit par nébulisation au moyen d'une buse à deux corps ou par un générateur d'aérosol, de préférence selon la nébulisation par ultrasons.

3. Utilisation des oxydes pyrogènes dopés au moyen d'un aérosol selon la revendication 1,
comme charge, comme support, comme substance catalytiquement active, comme produit de départ pour la production de dispersions, comme matériau de polissage pour le polissage de disques de métal ou, selon les cas, de silicium dans l'industrie électrique, comme matière de base céramique, dans l'industrie électronique (applications CMP), dans l'industrie des cosmétiques, comme additif dans l'industrie des silicones et des caoutchoucs, pour le réglage de la rhéologie des systèmes liquides, pour la stabilisation thermique, dans l'industrie des vernis, et comme matériau isolant thermique.

4. Dispositif pour réaliser le procédé selon la revendication 2,
caractérisé en ce qu'
on dispose dans un brûleur du type connu pour la production d'oxydes pyrogènes, pour l'introduction de l'aérosol, un tube supplémentaire, de préférence axial, le tube se terminant avant la buse du brûleur.
